# EUROPÄISCHE PATENTSCHRIFT

(11) **EP 0 378 756 B1**
(45) Veröffentlichungstag und Bekanntmachung des Hinweises auf die Patenterteilung: **04.08.1993**
(21) Anmeldenummer: 89119446.6
(22) Anmeldetag: 20.10.1989
(51) Int. Cl.: C07C 29/136, C07C 31/20, C07C 31/27

(54) **Verfahren zur Herstellung von aliphatischen und cycloaliphatischen Diolen durch katalytische Hydrierung von Dicarbonsäureestern**
Process for the preparation of aliphatic and cycloaliphatic diols by the catalytic hydrogenetion of dicarboxylic-acid esters
Procédé de préparation de diols aliphatiques et cyclo-aliphatiques par hydrogénation catalytique d'esters d'acides dicarboxyliques

(30) Priorität: 24.12.1988 DE 3843956
(43) Veröffentlichungstag der Anmeldung: 25.07.1990
(73) Patentinhaber: HÜLS AKTIENGESELLSCHAFT, 45764 Marl (DE)
(72) Erfinder: Fuhrmann, Werner, Dr., D-4358 Haltern (DE); Bub, Günther, Dr., D-4370 Marl (DE); zur Hausen, Manfred, Dr., D-4370 Marl (DE)

(56) Entgegenhaltungen:
- EP-A- 0 143 634
- DE-A- 2 719 867
- DE-B- 1 144 703
- US-A- 2 091 800

## Beschreibung

Cycloaliphatische und aliphatische alpha,omega-Diole werden technisch durch Hydrierung der entsprechenden Dicarbonsäureester hergestellt. Als Katalysatoren für die Hydrierung werden vorzugsweise Kupfer-Katalysatoren, gegebenenfalls mit Chrom und Barium als Promotoren, sowohl mit als auch ohne Träger verwendet. Als weitere Promotoren kommen neben dem Erdalkalimetall Ba auch Alkalimetalle wie Na und K als Promotoren zum Einsatz. Die nit diesen Katalysatoren sowohl in der Sumpf-als auch in der Rieselphase durchgeführten Hydrierungen lassen es allerdings nicht zu, bei hohen Umsätzen gleichzeitig hohe Selektivitäten für das gewünschte Diol zu erzielen. Dadurch wird eine destillative Aufarbeitung des Hydrieraustrages nötig, um höher als Diole siedende Komponenten abzutrennen.

So wird z. B. in der EP-PS 55 die Herstellung eines entsprechenden Hydrierkatalysators bzw. dessen Verwendung beschrieben, wobei man wäßrige Lösungen von Salzen des Kupfers, Nickels und/oder Kobalts mit Alkalisilikatlösungen in einem bestimmten Verhältnis vermischt und der Metallgehalt an Kupfer, Nickel und/oder Kobalt im fertigen Katalysator 40 bis 80 Gew.-% beträgt.
Im Anwendungsbeispiel 1 wird mit einem Cu auf SiO₂-Katalysator, der mit 5 Gew.-% in einem Gemisch von C₄-C₆-Dicarbonsäuremethylestern suspendiert ist, bei T̅ = 230 °C und P = 250 bar zu den entsprechenden Diolen hydriert. Nach 12 Stunden Reaktionszeit ist die ursprüngliche Esterzahl von 700 lediglich auf 60 zurückgegangen.

Gemäß der DE-PS 26 11 374 werden Katalysatoren für die Hydrierung eines Carbonsäureesters zu dem der Carbonsäure entsprechenden Al kohol beansprucht, die aus Kupfer-, Chrom- und Alkaliverbindungen und gegebenenfalls einer Erdalkaliverbindung sowie gegebenenfalls einem Träger bestehen. Dabei müssen je Grammatom Kupfer 0,3 bis 0,7 Grammatom Chrom vorhanden sein. So wird zur Hydrierung eines Adipinsäure-hexandiolesters, gelöst im Verhältnis 1 : 3 in Hexandiol-(1.6), ein trägerfreier Cu-CrNa-Katalysator verwendet. Bei T̅ = 200 °C,P = 250 bar, einer Katalysatorbelastung von 0,4 kg Gemisch/l Reaktionsraum x h und einem Wasserstoffdurchsatz von 50 Nl H₂/l Reaktionsraum x h werden in einem Hydrierreaktor zwar praktisch vollständiger Umsatz erreicht, bezogen auf die umgesetzte Dicarbonsäure neben 0,5 bis 1 % Destillationsrückstand aber nur 98,5 bis 99 % Hexandiol-1.6 erhalten.

Weiterhin werden nach DE-PS 11 54 448 höhermolekulare Alkohole durch katalytische Hydrierung von Fettsäureestern mittels Kupfer-Mehrstoff katalysatoren hergestellt. Beispielsweise wird nach Beispiel 3 Adipinsäure-di-n-butylester bei einer Katalysatorbelastung von 0,27 kg Ester/l x h und einer Gasbelastung von 10 570 Nl/l Kat. x h an einem ebenfalls trägerfreien CuZnCr-Katalysator bei T̅ = 250 °C und P = 220 bar zu Hexandiol-(1.6) hydriert. Bei fast vollständigem Umsatz (V.Z. < 3) wird nach Destillation eine Ausbeute von 90 bis 95 % ermittelt. Unter denselben Bedingungen wird Sebazinsäure-di-n-butylester zu Decandiol-(1.10) hydriert. Es wird eine Ausbeute von 90 bis 95 % der Theorie erzielt.

Gemäß DE-PS 11 59 925 wird ein Kupfer-Chrom-Kalium-Hydrierkatalysator auf einem Kieselsäuregelträger beschrieben, wobei man eine weitpori ge Kieselsäure mit bestimmter aktiver Oberfläche mit den entsprechenden Verbindungen tränkt und trocknet, und wobei der fertige Katalysator 18 bis 30 Gew.-% Kupfer, 0,3 bis 3,5 Gew.-% Chrom und 0,5 bis 9 Gew.-% Kalzium enthält.
Nach Beispiel 1 c wird bei T̅ = 215 bis 222 .C, P = 200 bar, einer Katalysatorbelastung von 0,1 kg Adipinsäure-di-ethylhexylester/l Kat. x h und einer Gasbelastung von 282 Nl H₂/l Kat. x h an einem CuCrK auf SiO₂-Trägerkatalysator Hexandiol-(1.6) nach Destillation bei fast vollständigem Umsatz (V.Z. = 2) mit einer Ausbeute von 94,5 % der Theorie erhalten. Die Hydrierung von Sebazinsäure-di-n-butyl ester nach Beispiel 1 f bei T̅ = 220 bis 225 °C mit sonst gleichen Bedingungen ergibt Decandiol-(1.10) nach Destillation bei fast vollständigem Umsatz (V.Z. = 3) mit einer Ausbeute von 96 % der Theorie. Die Hydrierung von Hexahydroterephthalsäure-di-n-butylester nach Beispiel 1 d bei T̅ = 210 °C, einer Gasbelastung von 28,2 Nl H₂/l Kat. x h unter sonst gleichen Bedingungen ergibt bei ebenfalls fast vollständigem Umsatz (V.Z. = 1) nach Destillation 1.4-Dimethylolcyclohexan mit einer Ausbeute von 95 % der Theorie.

In der DE-PS 12 02 269 wird die Herstellung von 1,4-Dimethylolcyclohexan durch katalytische Hydrierung eines Terephthalsäuredialkylesters zu einem Hexahydroterephthalsäuredialkylester in erster Stufe an einem Palladiumkatalysator und in zweiter Stufe die weitere Hydrierung des Hexahydroterephthalsäuredialkylesters an einem Kupferchromitkatalysator beschrieben. Hierfür geeignete Katalysatoren enthalten Kupfer entsprechend 30 bis 80 % CuO, Chrom entsprechend 15 bis 55 % Cr₂O₃ sowie einen Bariumgehalt entsprechend 0 bis 15 % BaO.
In den Beispielen wird die Hydrierung des Hexahydroterephthalsäure-dimethylesters an einem Kupferchromit-Trägerkatalysator mit folgender Zusammensetzung durchgeführt:
33,2 Gew.-% Cu als CuO, 38,0 Gew.-% Cr als Cr₂O₃, 10,4 Gew.-% Ba als BaO, 3,5 Gew.-% als Na₂O und 9,5 Gew.-% SiO₂.

Für die bei T̅ = 260 bis 270 °C und ca. P = 380 bis 390 bar durchgeführte Esterhydrierung werden ein Umsatz von ca. 97 % und ein Gehalt von 68 Gew.-% 1.4-Dimethylcyclohexan im flüssigen Reaktionsaustrag an gegeben. Entsprechend wird für die bei gleichen Bedingungen durchgeführte Hydrierung des Hexahydroterephthalsäure-di -butylesters praktisch vollständiger Umsatz und ein Gehalt von 49,1 Gew.-% 1.4-Dimethylcyclohexan im flüssigen Reaktionsaustrag angegeben. Da keine Angaben zur Menge der jeweils gebildeten gasförmigen C-Verbindungen gemacht werden, beträgt die maximale Ausbeute für den Methylester 94,4 % d. Th. und für den Butylester 96,8 % d. Th. Der verwendete Katalysator verliert wegen der eingestellten scharfen Reaktionsbedingungen nach 2 Monaten Laufzeit an Aktivität.

In der DE-AS 1 144 703 wird ein Verfahren zur Herstellung von Alkoholen durch Hydrierung von Estern in Gegenwart eines Katalysators beschrieben, wobei der Katalysator Kupfer entsprechend 30 bis 80 % CuO, Chrom entsprechend 15 bis 55 % Cr₂O₃, Barium entsprechend 0 bis 15 % BaO und 2 bis 25 % eines wasserlöslichen Bindemittels enthält. Es handelt sich um eine diskontinuierliche Sumpfphasenhydrierung in einem Schüttelautoklaven, Selektivitäten/Ausbeuten sind bei gleichzeitig unvollständigem Umsatz gering.

Gemäß EP-A 143 634 wird ein spezielles Hydrierverfahren zur Herstellung von Butandiol-1,4 beansprucht. Die Hydrierung erfolgt in der Gasphase mit Cu/Cr-haltigen Katalysatoren, Ausbeuten/Selektivitäten sind gering.

Die DE-OS 27 19 867 zeigt ein spezielles Verfahren zur Herstellung von Bernsteinsäure-di-n-butylester - aus Acrylnitril über Bernsteinsäuredinitril - und dessen Hydrierung zu Butandiol-1.4.

Nach US-PS 2 091 800 werden mit Cu/Cr-haltigen Katalysatoren ebenfalls Hydrierungen von Estern durchgeführt, wobei sich aber stets eine destillative Aufarbeitung anschließt.

Die bisher bekannten Verfahren zur katalytischen Hydrierung von Dicarbonsäureestern zur Herstellung von alpha,omega-Diolen weisen demnach Nachteile, wie das Erfordernis vergleichsweise scharfer Reaktionsbedingungen, das Nachlassen der Katalysatoraktivität bei längerer Betriebszeit und das Erfordernis einer destillativen Aufarbeitung auf, da sich Höhersieder bilden (DE-PS 26 11 376).

Es stellt sich daher die Aufgabe, ein Verfahren zur Herstellung von alpha,omega-Diolen durch Hydrierung von Dicarbonsäureestern zu finden, das diese Nachteile vermeidet und die hochselektive Bildung der daraus herstellbaren Diole ohne Bildung von Höhersledern und anderen Rückständen bei gleichzeitig milden Reaktionsbedingungen zur Erzielung großer Standzeiten der eingesetzten Katalysatoren erlaubt.

Gegenstand der Erfindung ist daher ein Verfahren zur Herstellung aliphatischer und cycloaliphatischer alpha,omega-Diole durch katalytische Hydrierung von Dicarbonsäureestern in Gegenwart von Wasserstoff mittels eines Kupferchromitkatalysators mit einem Kupfer- bzw. Chromgehalt, berechnet als CuO bzw. Cr₂O₃, im Bereich von 40 bis 47 Gew.-% und einem Bariumgehalt, berechnet als BaO, von bis zu 10 Gew.-%, welches dadurch gekennzeichnet ist, daß man Dicarbonsäureester mit oder ohne Lösemittel bei Temperaturen von 120 bis 220 °C und Drücken von p = 50 bis 400 bar in Rieselfahrweise in Gegenwart von Wasserstoff hydriert und aus dem erhaltenen rohen Hydrieraustrag das Diol als reines Sumpfprodukt ohne Überkopf-Destillation gewinnt, wobei der Katalysator eine spezifische Oberfläche von 20 bis 40 m²/g, ein Porenvolumen von 0,20 bis 0,24 cm³/g und ein Schüttgewicht von 1,2 bis 1,6 g/cm³ besitzt.

Erfindungsgemäß bringt man den Dicarbonsäureester bei erhöhter Temperatur im Bereich von 120 bis 220 °C in Gegenwart von Wasserstoff bei einem Druck von 50 bis 400 bar mit einem speziellen Kupferchromit-Katalysator in Kontakt. Die Flüssigkeitsbelastung des Katalysators liegt im allgemeinen Bereich von 0,1 bis 0,7 m³/m³ Kat. x h, die Gasbelastung im Bereich 1 000 bis 5 000 Nm³/m³ Kat. x h. Neben der Reaktionsführung mittels Rieselfahrweise (mit gasgefülltem Reaktor unter geringer Flüssigkeitsbelastung) ist auch eine Reaktionsführung mittels Sumpffahrweise (mit flüssigkeitsgefülltem Reaktor unter geringer Gasbelastung) möglich, jedoch wird die Rieselfahrweise bevorzugt.

Die Ester können entweder in reiner oder gelöster Form hydriert werden. Insbesonders wenn die eingesetzten Ester bei Umgebungstemperatur in fester Form vorliegen, empfiehlt es sich, die Ester mit einem bei Umgebungstemperatur flüssigen Lösemittel zu vermischen. Vorzugsweise verwendet man dabei als Lösemittel den bei der Esterhydrierung entstehenden Alkohol. Selbstverständlich müssen sich die verwendeten Lösemittel unter Hydrierbedingungen inert verhalten.

Die verwendeten Katalysatoren haben einen Kupfer- bzw. Chromgehalt (berechnet als CuO bzw. Cr₂O₃) im Bereich von 40 bis 47 Gew.-%; bevorzugt wird ein Gehalt an Kupfer und Chrom von jeweils 44 Gew.-%. Der Bariumgehalt (berechnet als Oxid) der verwendeten Katalysatoren beträgt bis zu 10 Gew.-%; bevorzugt wird ein Gehalt von 9 Gew.-%. Die physikalischen Größen der verwendeten Kupfer-Chromitkatalysatoren liegen für die spezifische Oberfläche im Bereich 20 bis 40 m²/g, für das Porenvolumen im Bereich 0,20 bis 0,24 cm³/g und für das Schüttgewicht im Bereich 1,2 bis 1,6 g/cm³. Die äußere Form der Katalysatoren ist durch gewählte Reaktionsführung festgelegt.

Die Aufarbeitung des Hydrieraustrages kann aufgrund der hochselektiven Reaktionsführung unter Verzicht auf die bislang notwendige und ebenso kostenintensive Destillation des Diols erfolgen. Es hat sich überraschenderweise gezeigt, daß die Bildung von höhersiedenden Nebenprodukten unter den erfindungsgemäß angewandten Hydrierbedingungen so gering ist, daß sich die Aufarbeitung des Diols auf ein Abtreiben des Lösemittels bzw. des bei der Esterhydrierung entstandenen Alkohols sowie auf die Entfernung der verfahrensspezifisch in kleinen Mengen gebildeten Leicht- und Zwischensieder, z. B. durch Wasserdampf-Schleppdestillation aus der Lösung des rohen Hydrieraustrages, beschränken kann. Es wird bei Temperaturen von 50 bis 200 °C, bevorzugt 100 bis 150 °C, und Drücken von 2 bis 500 mbar, bevorzugt 10 bis 160 mbar, gearbeitet. Zur Feinreinigung des vom Lösemittel oder vom Alkohol und von Leicht-und Zwischensiedern bereits weitestgehend befreiten Diols wird das Produkt anschließend einer Wasserdampfbehandlung unter ebenfalls sehr milden Bedingungen unterworfen und im Vakuum getrocknet. Als Sumpfprodukt der Aufarbeitung wird hochreines alpha,omega-Diol gewonnen.

Apparativ geeignet zur Verfahrensdurchführung ist jeder beheizbare Rührkessel ohne Destillationsaufsatz - evtl. mit Spritzschutz -, der unter Pumpenvakuum betrieben werden kann. Die Aufarbeitung kann kontinuierlich und/oder im Chargenbetrieb erfolgen.

Die folgenden Beispiele dienen zur näheren Erläuterung der Erfindung.

### Beispiel 1

In einen Rieselbettreaktor mit einem Volumen von 1 500 l werden 2 380 kg eines Kupferchromit-Katalysators mit einem Gehalt an Cu, berechnet als CuO von 44,9 Gew.-%, an Cr, berechnet als Cr₂O₃ von 45,8 Gew.-% und an Ba, berechnet als BaO von 9,1 Gew.-% sowie mit einem Gehalt an Al₂O₃, SiO₂ und SrO von 0,2 Gew.-%, eingefüllt. Nach der Reduktion des Katalysator mit einem H₂/N₂-Gemisch werden eine Flüssigbelastung von 0,23 m³ Adipinsäure-di-n-butylester/m³ Katalysator x h, eine Gasbelastung von 3 670 Nm³ H₂/m³ Katalysator x h und eine mittlere Reaktionstemperatur über die Bettlänge von T̅ = 182 °C eingestellt. Im Produktaustrag wird eine Esterzahl von 0,15 gemessen. Nach 10 Betriebstagen hat sich eine konstante Aktivität des Katalysators bei einer Belastung von 0,2 m³ Ester/m³ Katalysator x h und einer mittleren Katalysatortemperatur über die Bettlänge von T̅ = 192 °C bei einem Druck von P = 300 bar eingestellt. Die sich bei diesen Bedingungen im Produktionsaustrag einstellende Esterzahl liegt zwischen 0,1 bis 0,2 mg KOH/g. Die Säurezahl liegt bei Null. Eine GC-MS-Analyse des Produktaustrages zeigt neben dem dieser Esterzahl entsprechenden Estergehalt lediglich Spuren im Bereich 3 000 ppm von höher als Hexandiol-(1.6) siedenden Komponenten (Ethern) an.

Nach 40 Betriebstagen sind 284 t Ester hydriert worden. Aktivität und Selektivität des Katalysators sind konstant geblieben.

Der durch Hydrierung des Adipinsäure-di-n-butylesters zusammen mit zugesetztem n-Butanol erhaltene Roh-Hexandiol-(1.6)-Austrag enthält 40,97 Gew.-% Hexandiol-(1.6), 58,43 Gew.-% n-Butanol sowie in Summe 0,60 Gew.-% gaschromatographisch nachgewiesenen Vor-, Zwischenlauf und Rest. Der Hydrieraustrag wird kontinuierlich in einen 35 m³-Rührkessel übernommen. Die Übernahme erfolgt in dem Maße, wie n-Butanol bei 125 °C und 50 mbar abdestilliert, der Hexandiol-(1.6)-Gehalt im Destillat beträgt hierbei 0,3 Gew.-%.

Nach Übernahme von etwa 50 t der butanolischen Hexandiol-(1.6)-Lösung wird der Zulauf zum Reaktor gestoppt. Bei Erreichen eines Rest-Butanol-Gehaltes < 1 Gew.-% des Sumpfes wird der Kesselinhalt durch Eindüsen von Wasser einer Wasserdampfbehandlung unterworfen. Hierbei werden innerhalb 5 Stunden 5 Gew.-% Wasser, bezogen auf die Hexandiol-(1.6)-Menge, eingeleitet, wobei die Bedingungen 115 bis 120 °C und 65 mbar betragen. Die bei der Wasserdampfbehandlung anfallende Destillatmenge, die wasserdampfflüchtigen Anteile von Hexandiol-(1.6) in einer Menge von 1,5 Gew.-%, bezogen auf Hexandiol-(1.6)-Einsatz, enthält, wird separat erfaßt, gesammelt und einer späteren Aufarbeitung zugeführt. Das Sumpfprodukt wird bei 110 bis 115 °C und 10 mbar getrocknet, es stellt hochreines Hexandiol-(1.6) mit einem Gehalt von > 99,9 Gew.-% dar. Die weiteren Kenngrößen des Produktes sind: Erstarrungspunkt > 42 .C, Wassergehalt < 0,1 Gew.-%, Farbe = 5 APHA, Säurezahl < 0,01 mg KOH/g.
Die Ausbeute über alle Stufen beträgt 75,19 kg Hexandiol-(1.6) pro 100 kg Adipinsäure-Einsatz (= 92,98 % der Theorie).

### Beispiel 2

In einen Rieselbettreaktor mit einem Volumen von 5 000 l werden 7 140 kg des in Beispiel 1 beschriebenen Kupferchromit-Katalysators eingefüllt. Nach der Reduktion des Katalysators mit einem H₂/N₂-Gemisch werden bei der Hydrierung von Sebazinsäure-di-n-butylester nach Erreichen des stationären Zustandes eine Flüssigbelastung von 0,16 m³ Ester/n-Butanol -Gemisch/m³ Katalysator x h, eine Gasbel astung von 4 000 Nm³ H₂/m³ Katalysator x h und eine mittlere Katalysatortemperatur über die Bettlänge von T̅ = 185 °C bei einem Druck von P = 300 bar eingestellt. Im Produktaustrag wird eine Esterzahl zwischen 0,1 bis 0,25 mg KOH/g gemessen. Die Säurezahl liegt bei Null. Eine GC-MS-Analyse des Produktaustrages zeigt neben dem dieser Esterzahl entsprechenden Estergehalt höher als Decandiol-(1.10) siedende Komponente im Bereich 2 000 bis 4 000 ppm an. Aktivität und Selektivität des Katalysators sind konstant.

Der durch Hydrierung des Sebazinsäure-di-n-butylesters zusammen mit zugesetztem n-Butanol erhaltene Roh-Decandiol-(1.10)-Austrag enthält 67,71 Gew.-% n-Butanol, 31,27 Gew.-% Decandiol-(1.10) sowie in Summe 1,02 Gew.-% an Vor-, Zwischenlauf und Rest. Das rohe Decandiol-(1.10) wird entsprechend dem Beispiel 1 zur Aufarbeitung übernommen und behandelt. Die Betriebsbedingungen bis zum Erreichen eines Rest-Butanol-Gehaltes < 0,5 Gew.-% des Sumpfes sind max. 130 °C und 40 mbar. Der Sumpf wird anschließend der Wasserdampfbehandlung mit 5 % Wasser (bezogen auf Decandioleinsatz) unterworfen; dabei werden eine Temperatur von T̅ = 135 °C und ein Druck von p = 60 mbar für 30 Minuten eingestellt. Danach wird bei 150 °C und 20 mbar getrocknet, wobei der Wasserwert nach 1 Stunde < 0,1 Gew.-% beträgt. Das Sumpfprodukt stellt hochreines Decandiol-(1.10) mit einem Gehalt > 99,3 Gew.-% dar. Die weiteren analytischen Daten des Produkts sind: Farbe = 10 APHA; Säurezahl < 0,01 mg KOH/g; Erstarrungspunkt = 73 °C.

Das Rückbutanol aus der Abtoppung ist 99,4 %ig und enthält neben geringen Mengen Vor- und Zwischenlauf etwa 0,1 Gew.-% Decandiol-(1.10). Das Destillat der Wasserdampfbehandlung enthält Decandiol-(1.10) in einer Menge von 3 Gew.-%, bezogen auf Decandiol-(1.10)-Einsatz.

### Beispiel 3

In einen Rieselbettreaktor mit einem Volumen von 8 000 l werden 12 066 kg des in Beispiel 1 beschriebenen Kupferchromit-Katalysators eingefüllt, um Adipinsäure-di-n-butylester zu hydrieren. Nach Reduktion des Katalysators und Erreichen des stationären Zustandes wird eine Flüssigbelastung von 0,16 m³ Ester/m³ Katalysator x h, eine Gasbelastung von 2 000 Nm³ H₂/m³ Katalysator x h und eine mittlere Katalysatortemperatur über die Bettlänge von T̅ = 170 °C eingestellt.

Insgesamt werden in 63 Hydriertagen 1 612 t Ester über den Katalysator gefahren, wobei der Durchsatz von 1 250 l Ester/h auf 1 300 l Ester/h gesteigert und die mittlere Katalysatortemperaturvon T = 170 °C auf T̅ = 157 °C abgesenkt wird. Der Roh-Hexandiol-(1.6)-Austrag hat über die gesamte Hydrierkampagne die bereits im Beispiel 1 genannte Zusammensetzung; die Esterzahl liegt bei 0,1 mg KOH/g.

Die Aufarbeitung erfolgt wieder auf die gleiche Weise und mit dem gleichen Ergebnis wie in Beispiel 1 bereits beschrieben. Ober alle Stufen beträgt der Einsatzfaktor 1,33 t Adipinsäure/t Hexandiol-(1.6).

### Beispiel 4

Im Anschluß an die im vorangegangenen Beispiel beschriebene Hexandiol-(1.6)-Kampagne wird bei den gleichen Bedingungen wie in Beispiel 3 nach sorgfältiger Spülung Hexahydroterephthalsäuredimethylester hydriert.

Die Hydrierung wird mit einem Flüssigkeitsdurchsatz von 1 000 l Ester/h, einer Kreisgasmenge von 18 000 Nm³/h bei einer mittleren Katalysatortemperatur von T̅ = 190 °C durchgeführt. Die Esterzahl im Reaktionsaustrag liegt ebenso wie die Säurezahl bei Null, die höher als die beiden 1.4-Cyclohexandimethanol-Isomere siedenden Komponenten liegen im Spurenbereich.

164 t Rohaustrag, der zusammen mit zugesetztem Methanol 62 Gew.-% 1.4-Cyclohexandimethanol enthält, wird chargenweise in mehreren Partien zur Aufarbeitung in einen 35 m³-Rührbehälter übernommen. Bei Temperaturen im Kessel, die während der Chargendauer allmählich bis 100 °C angehoben werden und einem gleichzeitig von 160 bis 90 mbar verbesserten Vakuum wird Methanol abdestilliert. Bei Erreichen eines Rest-Methanol-Gehaltes von 0,75 Gew.-% des Sumpfes wird auf 150 °C aufgeheizt. Bei dieser Temperatur sowie einem Vakuum von 120 mbar werden während 5 1/2 Stunden insgesamt 1 800 l Wasser in das Produkt eingespritzt. Anschließend wird 3 1/2 stunden bei gleicher Temperatur und gleichem Druck getrocknet.

Das Sumpfprodukt stellt hochreines 1.4-Cyclohexandimethanol mit einem Gehalt > 99,9 Gew.-% dar. Die weiteren Kenngrößen des Produktes sind: Erstarrungspunkt > 33 °C; Wassergehalt < 0,1 Gew.-%; Farbe = 5 bis 10 APHA; Säurezahl < 0,01 mg KOH/g.

## Patentansprüche

1. Verfahren zur Herstellung aliphatischer und cycloaliphatischer alpha,omega-Diole durch katalytische Hydrierung von Dicarbonsäureestern in Gegenwart von Wasserstoff mittels eines Kupferchromitkatalysators mit einem Kupfer- bzw. Chromgehalt, berechnet als CuO bzw. Cr₂O₃, im Bereich von 40 bis 47 Gew.-% und einem Bariumgehalt, berechnet als BaO, von bis zu 10 Gew.-%,
dadurch gekennzeichnet,
daß man Dicarbonsäureester mit oder ohne Lösemittel bei Temperaturen von 120 bis 220 °C und Drücken von p = 50 bis 400 bar in Rieselfahrweise in Gegenwart von Wasserstoff hydriert und aus dem erhaltenen rohen Hydrieraustrag das Diol als reines Sumpfprodukt ohne ÜberkopfDestillation gewinnt, wobei der Katalysator eine spezifische Oberfläche von 20 bis 40 m²/g, ein Porenvolumen von 0,20 bis 0,24 cm³/g und ein Schüttgewicht von 1,2 bis 1,6 g/cm³ besitzt.

2. Verfahren nach Anspruch 1,
dadurch gekennzeichnet,
daß der Kupferchromit-Katalysator einen Kupfer- bzw. Chromgehalt von jeweils 44 Gew.-% und einen Bariumgehalt von 9 Gew.-% besitzt.

3. Verfahren nach den Ansprüchen 1 und 2,
dadurch gekennzeichnet,
daß Lösemittel und/oder bei der Hydrierung entstehende Alkohole aus dem Hydrieraustrag durch Abtoppen entfernt werden.

4. Verfahren nach den Ansprüchen 1 und 2,
dadurch gekennzeichnet,
daß verfahrensspezifisch gebildete Leicht- und Zwischensieder durch Wasserdampf-Schleppdestillation aus dem Hydrieraustrag entfernt werden.

5. Verfahren nach den Ansprüchen 1 bis 4,
dadurch gekennzeichnet,
daß zur Feinreinigung des Diols eine Wasserdampfbehandlung durchgeführt wird.

6. Verfahren nach den Ansprüchen 3 bis 5,
dadurch gekennzeichnet,
daß bei Temperaturen von 50 bis 200 °C gearbeitet wird.

7. Verfahren nach Anspruch 6,
dadurch gekennzeichnet,
daß bei Temperaturen von 100 bis 150 °C gearbeitet wird.

8. Verfahren nach den Ansprüchen 3 bis 7,
dadurch gekennzeichnet,
daß im Vakuum bei 2 bis 500 mbar gearbeitet wird.

9. Verfahren nach Anspruch 8,
dadurch gekennzeichnet,
daß im Vakuum bei 10 bis 160 mbar gearbeitet wird.

## Claims

1. A method for the manufacture of aliphatic and cycloaliphatic alpha,omega-diols by catalytic hydrogenation of dicarboxylic acids in the presence of hydrogen by means of a copper chromite catalyst having copper and chromium contents, calculated as CuO and Cr₂O₃, respectively, in the range of 40 to 47% by weight and a barium content calculated as BaO of up to 10% by weight, characterized in that a dicarboxylic acid ester with or without solvent is hydrogenated in a trickling process at temperatures of 120 to 220°C and pressures of p = 50 to 400 bar in the presence of hydrogen and the diol is obtained from the crude hydrogenated product as a pure bottom product without top product distillation, and wherein the catalyst has a specific surface of 20 to 40 m²/g, a pore volume of 0.20 to 0.24 cm³/g and an apparent density of 1.2 to 1.6 g/cm³.

2. A method according to claim 1, characterized in that the copper chromite catalyst has copper and chromium contents of 44% by weight in each case and a barium content of 9% by weight.

3. A method according to claims 1 and 2, characterized in that the solvent and/or alcohols produced during the hydrogenation are removed from the hydrogenation product by top fraction distillation.

4. A method according to claims 1 and 2, characterized in that the process specific low and intermediate boiling compounds formed are removed from the hydrogenation product by steam entrainment distillation.

5. A method according to claims 1 to 4, characterized in that for fine purification of the diols a steam treatment is performed.

6. A method according to claims 3 to 5, characterized in that it is performed at temperatures of 50 to 200°C.

7. A method according to claim 6, characterized in that it is performed at temperatures of 100 to 150°C.

8. A method according to claims 3 to 7, characterized in that it is performed in a vacuum of 2 to 500 mbar.

9. A method according to claim 8, characterized in that it is performed in a vacuum of 10 to 160 mbar.

## Revendications

1. Procédé d'obtention d'α, ω,-diols aliphatiques et cycloaliphatiques par hydrogénation catalytique d'esters d'acides dicarboxyliques en présence d'hydrogène, à l'aide d'un catalyseur à la chromite de cuivre ayant une teneur en cuivre ou en chrome, calculée comme CuO ou Cr₂O₃, dans la zone de 40 à 47 % en poids et une teneur en baryum calculée comme BaO, allant jusqu'à 10 % en poids,
caractérisé en ce que l'on utilise l'hydrogènation d'un ester d'acide dicarboxylique avec ou sans solvant, à des températures allant de 120 à 220°C et à des pressions de p = 50 à 400 bars dans un mode de conduite par ruissellement en présence d'hydrogène et on obtient à partir du résidu brut d'hydrogénation, le diol sous forme de produit de bas de colonne pur sans distillation des toutes premières fractions, le catalyseur possèdant une surface spécifique allant de 20 à 40 m²/g, un volume de pores allant de 0,20 à 0,24 cm³/g et une densité apparente de 1,2 à 1,6 g/cm³.

2. Procédé selon la revendication 1, caractérisé en ce que le catalyseur à la chromite de cuivre possède une teneur en cuivre ou en chrome de - respectivement - 44 % en poids et une teneur en baryum de 9 % en poids.

3. Procédé selon les revendications 1 et 2, caractérisé en ce que le solvant et/ou les alcools qui sont formés au cours de l'hydrogénation sont éliminés du résidu d'hydrogénation par distillation primaire.

4. Procédé selon les revendications 1 et 2, caractérisé en ce que les substances qui bouillent facilement et les substances qui bouillent à des températures intermédiaires formées spécifiquement au procédé, sont chassées par distillation d'entraînement à la vapeur d'eau, du dégagement d'hydrogénation.

5. Procédé selon les revendications 1 à 4, caractérisé en ce que l'on effectue un traitement à la vapeur d'eau en vue de la purification fine du diol.

6. Procédé selon les revendications 3 à 5, caractérisé en ce que l'on travaille à des températures allant de 50 à 200°C.

7. Procédé selon la revendication 6, caractérisé en ce que l'on travaille à des températures allant de 100 à 150°C.

8. Procédé selon les revendications 3 à 7, caractérisé en ce que l'on travaille sous vide de 2 à 500 mbars.

9. Procédé selon la revendication 8, caractérisé en ce que l'on travaille sous vide de 10 à 160 mbars.
